# EUROPÄISCHE PATENTANMELDUNG

(11) **EP 4 309 664 A1**
(43) Veröffentlichungstag der Anmeldung: **24.01.2024**
(21) Anmeldenummer: 22750107.9
(22) Anmeldetag: 25.01.2022
(51) Int. Cl.: A61K 36/21, C12M 3/06, B01D 61/14, B03B 5/00

(54) **VERFAHREN ZUR ISOLIERUNG VON MIKROVESIKELN AUS PFLANZEN DER AMARANTHACEAE-FAMILIE**

(30) Priorität: 04.02.2021 RU 2021102559
(71) Anmelder: Gordeychuk, Vladimir Evgenevich, g. Krasnodar, 350001 (RU); Rubtsov, Sergey Vladimirovich, Moscow 109316 (RU); Barsukov, Aleksandr Pavlovich, Rostov-na-Donu 344113 (RU)
(72) Erfinder: ABRAMOV, Aleksandr Andreevich, Moscow, 119634 (RU); PETKEVICH, Alisa Antonovna, Moscow, 109651 (RU); POSPELOV, Vadim Igorevich, Kolomna, 140407 (RU)
(74) Vertreter: Rentsch Partner AG
(86) Internationale Anmeldenummer: PCT/RU2022/000020
(87) Internationale Veröffentlichungsnummer: WO 2022/169383

(57) **Zusammenfassung**

Die Erfindung betrifft Verfahren zur Isolation biologisch aktiver Stoffe aus Pflanzenrohstoff und insbesondere Verfahren zum Erhalt von Mikrovesikeln (mit einem Durchmesser von 50-1200 nm) aus Pflanzen der Familie der Fuchsschwanzgewächse. Das beanspruchte Verfahren kann in der Medizin-, Kosmetik- und Lebensmittelindustrie angewendet werden. Das technische Ergebnis des von den Erfindern bereitgestellten Verfahrens zur Isolation von Mikrovesikeln besteht in der Möglichkeit der Isolation von Mikrovesikeln aus Pflanzen der Familie der Fuchsschwanzgewächse. Das vorstehende technische Ergebnis wird durch Anwendung eines Verfahrens zur Isolation von Mikrovesikeln erzielt, umfassend das Sammeln von Pflanzen aus der Familie der Fuchsschwanzgewächse, die bis zum Erhalt eines homogenisierten Gemischs zerkleinert werden, von dem die flüssige Phase anschließend mittels eines Grobfilters getrennt wird, die im Anschluss über 30 Minuten hinweg bei 3.000 g bzw. über 60 Minuten hinweg bei 10.000 g zentrifugiert wird, anschließend das Durchführen einer Ultrazentrifugation des abgetrennten Überstands über 90 Minuten hinweg bei 150.000 g, anschließend das Suspendieren des erhaltenen Niederschlags in Natriumphosphatpuffer im Verhältnis von 1 : 10 und das erneute Ultrazentrifugieren über 90 Minuten hinweg bei 150.000 g, ferner das erneute Suspendieren des erhaltenen Niederschlags in Natriumphosphatpuffer im Verhältnis von 1 : 10, worauf die Durchführung einer Filtration mittels eines Filters mit einem Porendurchmesser von 1,2 µm folgt.

## Beschreibung

### BESCHREIBUNG DER ERFINDUNG

Verfahren zur Isolation von Mikrovesikeln aus Pflanzen der Familie der Fuchsschwanzgewächse.

Die Erfindung betrifft Verfahren zur Isolation biologisch aktiver Stoffe aus Pflanzenrohstoff und insbesondere Verfahren zum Erhalt von Mikrovesikeln (mit einem Durchmesser von 50-1200 nm) aus Pflanzen der Familie der Fuchsschwanzgewächse. Das beanspruchte Verfahren kann in der Medizin-, Kosmetik- und Lebensmittelindustrie eingesetzt werden.

Im Stand der Technik ist ein Verfahren zur Isolation von Mikrovesikeln aus Kulturmedium bekannt, das Zentrifugation, anschließende Ultrazentrifugation, Auflösung des erhaltenen Niederschlags in PBS und erneute Zentrifugation umfasst, s. S. 25, erste zwei Absätze unter dem Link https://www.researchgate.net/publication/323269152_Isolation_of_extracellular_ microvesicles_from_cell_culture_medium_Comparative_evaluation_of_methods.

Der größte Nachteil des unter Punkt 1 auf S. 25 angeführten Verfahrens besteht in der unzureichenden Effektivität, da das bekannte Verfahren nicht ermöglicht, dass Mikrovesikel der Größe nach von den übrigen Partikeln getrennt werden, die sich in dem erhaltenen Niederschlag befinden. Ein weiterer Nachteil besteht darin, dass als Ausgangsmaterial für die Isolation von Exosomen Zellkulturmedium verwendet wird, dessen Erhalt die Aufwendung bestimmter Reagenzien (Nährmedium, Serum, L-Glutamin, Natriumpyruvat, Antibiotika, Waschlösungen wie Salzlösung nach Hanks), die Bestellung und den Erhalt registrierter Zelllinien, die Einführung der erhaltenen Zelllinien oder den Erhalt der Zellkultur mit anschließender Notwendigkeit zur Beschreibung dieser Zellkultur erfordert. Die aufgezählten Etappen erfordern einen bestimmten materiellen und zeitlichen Aufwand, was das vorliegende Verfahren zum Erhalt von Mikrovesikeln mühsam und aufwändig macht.

Das technische Ergebnis des von den Erfindern bereitgestellten Verfahrens besteht in der Möglichkeit der Isolation von Mikrovesikeln aus Pflanzen der Familie der Fuchsschwanzgewächse.

Das vorstehende technische Ergebnis wird durch Anwendung eines Verfahrens zur Isolation von Mikrovesikeln erzielt, umfassend das Sammeln von Pflanzen aus der Familie der Fuchsschwanzgewächse, die bis zum Erhalt eines homogenisierten Gemischs zerkleinert werden, von dem die flüssige Phase anschließend mittels eines Grobfilters getrennt wird, die im Anschluss über 30 Minuten hinweg bei 3000 g bzw. über 60 Minuten hinweg bei 10000 g zentrifugiert wird, um das erhaltene Gemisch von großen Partikeln - Zellkonglomeraten und einzelnen Zellen - zu befreien, anschließend das Durchführen einer Ultrazentrifugation des abgetrennten Überstands über 90 Minuten hinweg bei 150000 g, anschließend das Suspendieren des erhaltenen Niederschlags in Natriumphosphatpuffer im Verhältnis von 1 : 10 suspendiert und das erneute Ultrazentrifugieren über 90 Minuten hinweg bei 150000 g. Die Etappen der Ultrazentrifugation sind erforderlich, um das Gemisch von apoptotischen Körperchen, Eiweißkonglomeraten und Eiweißen > 30 kDa zu befreien. Ferner wird der erhaltene Niederschlag erneut in Natriumphosphatpuffer im Verhältnis von 1 : 10 suspendiert, woraufhin eine Filtration mittels eines Filters mit einem Porendurchmesser von 1,2 µm durchgeführt wird, was eine Trennung der Mikrovesikel im gesuchten Spektrum - bis 1200 nm - von den übrigen Partikeln, die sich in dem erhaltenen Niederschlag befinden, ermöglicht. Darüber hinaus haben die Erfinder experimentell festgestellt, dass bei einer solchen Abfolge der Vorgänge und Abläufe das beanspruchte Verfahren ermöglicht, dass Mikrovesikel aus Pflanzen der Familie der Fuchsschwanzgewächse mit maximaler Effektivität isoliert werden.

Ausführungsbeispiel für das beanspruchte Verfahren:
Es werden 10 g Blätter, Blüten und Wurzeln (es sind beliebige Proportionen verwendbar, was sich nicht auf die Erzielung des beanspruchten technischen Ergebnisses auswirkt, da Mikrovesikel in den verschiedenen Pflanzenteilen in ungefähr gleichen Mengen vorhanden sind) von *Amaranthus-blitoides*-S.Wats.-Pflanzen in eine automatische Zerkleinerungsvorrichtung gegeben und bis zum Erhalt eines homogenen (gleichmäßigen) Gemischs zerkleinert. Anschließend wird die flüssige Phase mittels Filtration durch ein Sieb mit einem Porendurchmesser von 1-2 mm abgetrennt. Ferner wird eine anschließende Zentrifugation über 30 Minuten hinweg bei 3.000 g bzw. über 60 Minuten hinweg bei 10.000 g durchgeführt. Anschließend wird der erhaltene Niederschlag in Natriumphosphatpuffer im Verhältnis von 1 : 10 suspendiert und erneut über 90 Minuten hinweg bei 150.000 g ultrazentrifugiert, ferner wird der erhaltene Niederschlag erneut in Natriumphosphatpuffer im Verhältnis von 1 : 10 suspendiert, worauf die Durchführung einer Filtration mittels eines Filters mit einem Porendurchmesser von 1,2 µm folgt.

Anschließend wurden 500 µl erhaltene Lösung mit dem Verfahren der Photonenkorrelationsspektroskopie (Nanophox Sympatec GmbH, Deutschland) untersucht, bei dem Spektren im Bereich von 50-1.200 nm entdeckt wurden, was das Vorhandensein von Mikrovesikeln des angemeldeten Spektrums in der Lösung belegt.

## Patentansprüche

1. Verfahren zur Isolation von Mikrovesikeln, umfassend das Sammeln von Pflanzen aus der Familie der Fuchsschwanzgewächse, die bis zum Erhalt eines homogenisierten Gemischs zerkleinert werden, von dem anschließend die flüssige Phase mittels eines Grobfilters getrennt wird, die im Anschluss über 30 Minuten hinweg bei 3.000 g bzw. über 60 Minuten hinweg bei 10.000 g zentrifugiert wird, anschließend das Durchführen einer Ultrazentrifugation des abgetrennten Überstands über 90 Minuten hinweg bei 150.000 g, anschließend das Suspendieren des erhaltenen Niederschlags in Natriumphosphatpuffer im Verhältnis von 1 : 10 und das erneute Ultrazentrifugieren über 90 Minuten hinweg bei 150.000 g, ferner das erneute Suspendieren des erhaltenen Niederschlags in Natriumphosphatpuffer im Verhältnis von 1 : 10, worauf die Durchführung einer Filtration mittels eines Filters mit einem Porendurchmesser von 1,2 µm folgt.
